Europäisches Patentamt

European Patent Office

Office européen des brevets

(11) Publication number: **0 218 432**
A2

(12) **EUROPEAN PATENT APPLICATION**

(21) Application number: 86307438.1

(22) Date of filing: 29.09.86

(51) Int. Cl.⁴: **C 12 N 15/00**

(30) Priority: 30.09.85 JP 214869/85

(43) Date of publication of application:
15.04.87 Bulletin 87/16

(84) Designated Contracting States:
AT BE CH DE FR GB IT LI LU NL SE

(71) Applicant: Honjo, Tasuku
13-12, Ueno Higashi 3-chome
Tonyonaka-shi Osaka (JP)

(72) Inventor: Honjo, Tasuku
13-12, Ueno Higashi 3-chome
Tonyonaka-shi Osaka (JP)

(74) Representative: Cuddon, George Desmond et al
MARKS & CLERK Alpha Tower Suffolk Street Queensway
Birmingham B1 1TT (GB)

(54) Methods for cDNA cloning, and vector-primer DNA, plasmid DNA and linker DNA, used in the said methods.

(57) The present invention relates to the effective methods for cDNA cloning of a desired protein (called a target substance) by using a small amount of mRNA which is obtained from a cell generating and secreting the target substance and by tracing a biological activity of the target substance, in the case of not purified protein having a biological activity in a small amount.

EP 0 218 432 A2

**Description**

METHOD FOR cDNA CLONING, AND VECTOR-PRIMER DNA, PLASMID DNA AND LINKER DNA, USED IN THE SAID METHODS

BACKGROUND OF THE INVENTION

The present invention relates to methods for cdna cloning, and vector-primer DNA, plasmid DNA and linker DNA, used in the said methods. Particularly, the invention is useful in cDNA cloning of an unisolated protein, having a biological activity even in a small amount and it is extremely useful when only a small amount of messenger RNA (abbreviated as mRNA hereinafter) may be isolated from cells producing and secreting such protein.

Some substances controlling physiological function, such as lymphokines in immunological system, have a strong physiological activity in small amounts. When studying their function in more detail or applying them for therapeutic use, it is a very effective means to isolate the gene of the substance first.

Now, there are some well-known methods of cDNA cloning. When the said protein is purified to homogeneity, it is carried out by determining amino acid sequence in a part of the protein, synthesizing oligonucleotides corresponding to the amino acid sequence, and then selecting a clone hybridizing with the oligonucleotides, from complementary DNA (abbreviated as "cDNA" hereinafter) library (Pennica, D., et al., Nature, 312, 724, 1984).

When the said protein, having a biological activity, is impure because of trace amounts and some difficulties, there is a method of cloning by tracing the activity.

For example, in the case of cDNA cloning of interleukin 2, mRNA is separated from cells secreting interleukin 2, and a cDNA library is prepared from mRNA fractionated by the sucrose gradient method. By using this cDNA library, mRNA is selected by hybrid-formation method, and then the protein translated from the mRNA is assayed on the activity. The repetition of the above procedures gives a single cDNA (Taniguchi, T., et al., Nature, 302, 305, 1983). The said method requires at least several hundreds micrograms of mRNA.

On the other hand, cloning vector system which may allow cDNA to establish itself in an animal cell (Cos cell), has been developed (Okayama, H. and Berg, P., Mol. Cell. Biol., 3, 280, 1983). However, this system has the following defects. The efficiency in introducing vector DNA in a cell changes in each sample; the yield of protein changes largely according to the state of the cell; and it is difficult to assay on the activity because secreted protein, secreted outside the cell is diluted with a large amount of medium and contaminated by serum protein simultaneously, and non-secreted protein, not secreted outside the cell is degraded by a large amount of protease existing in Cos cell.

BRIEF SUMMARY OF THE INVENTION

The present inventor has improved the defects of such known methods for cDNA cloning and has established the effective methods for cDNA cloning of a desired protein (called a target substance) by using a small amount such as several micrograms, of several ten thousand or more kinds of mRNA which is obtained from a cell generating and secreting the target substance and by tracing a biological activity of the target substance, in the case of not purified protein having a biological activity in a small amount, having completed the present invention.

DETAILED DESCRIPTION OF THE INVENTION

Accordingly, the methods for cDNA cloning, of the present invention, is characterized by:

(a) isolating and fractionating a mRNA group containing mRNA of a target substance in a well-known method (called "conventional method").

(b) hybridizing the mRNA group with a vector primer DNA containing a deoxythymidine (abbreviated as "dT" hereinafter) chain at a position upstream from a polylinker obtained from a known plasmid DNA,

(c) synthesizing a single-chained cDNA by using reverse transcriptase and then inserting a deoxyribonucleotide chain at the end to obtain a vector DNA,

(d) linking up the vector DNA with a linker DNA which has a SP6 promoter followed by the deoxyribonucleotide chain which can make a pair with the deoxyribonucleotide chain inserted in the process (c) at a position downstream from the promoter obtained from a known plasmid DNA, and then replacing mRNA by DNA to obtain a plasmid DNA group corresponding to each mRNA in mRNA groups,

(e) transforming the host by using the plasmid DNA group thus obtained,

(f) separating the plasmid DNA group from the transformant, subjecting to in-vitro transcription using SP6 RNA polymerase and using as template, the cDNA group which is obtained by digesting with each of some kinds of restriction endonuclease, to synthesize mRNA groups, translating the mRNA group to obtain protein groups, and then assaying the protein groups on activity of the target substance to select a proper restriction endonuclease, and

(g) subjecting each groups containing a small number of the transformants obtained in the process (e), to the same procedure as the process (f) by using the restriction endonuclease selected in the process (f) to select an active group, and then subjecting the transformants of the selected group to the same procedure, to separate a single clone finally.

Process (a) may be carried out by separating poly A·RNA from cells producing or secreting a target substance in a conventional method, fractionating the obtaining poly A·RNA in a conventional method, e.g. by the sucrose gradient method, translating a part of each fraction in a conventional method, e.g. by injecting into oocytes of <u>Xenopus laevis</u>, and then screening the activity of the resulting proteins, to obtain a fraction containing mRNA of the target substance (mRNA group). The process is generally well-known.

Process (b) may be carried out by cleaving a known plasmid DNA such as pcDV1 and pUC8 by a proper restriction endonuclease in a conventional method, as it is if the plasmid DNA itself has a proper polylinker or after inserting a proper polylinker if it has not a proper polylinker, and then inserting dT chains at a position upstream from the polylinker, preferably about 50 dT chains at a position a long way upstream from the polylinker to obtain vector-primer DNA. Any polylinker may be inserted, and a polylinker having many recognition sites by restriction endonucleases may be preferably used. Insertion of polylinker and dT chains may be carried out by using known ligase and terminal transferase, respectively, in a conventional method. The obtained vector-primer DNA may be hybridized with mRNA group obtained in the procedure (a) in a conventional method.

Process (c) is a known method for synthesizing DNA, using RNA as template and using a known reverse transcriptase, and to the end of a single cDNA thus obtained, deoxyribonucleotide chain may be added in the same procedure as described in the procedure (b), to obtain vector DNA. As the number of deoxyribonucleotide chains to be added, 10 to 15 deoxycytidine (abbreviated as dC hereinafter) chains are preferred.

Process (d) may be carried out by the following procedures; a known plasmid DNA having a SP6 promoter such as pSP-62-PL and pSP64 may be digested with a proper restriction endonuclease in a conventional method at a position downstream from the SP6 promoter, preferably at a very position downstream it, and the deoxyribonucleotide chain which can make a pair with the deoxyribonucleotide chain inserted in the process (c), may be inserted at the digesting position, and then it may be digested at a position upstream from the SP6 promoter to obtain a linker DNA. The linker DNA thus obtained may be linked up with the vector DNA obtained in the procedure (c) by using a known DNA ligase, and then a mRNA may be replaced by a DNA to obtain a plasmid DNA group corresponding to each mRNA in mRNA groups. Each process abovementioned may be carried out by well-known methods.

Process (e) may be carried out by transforming a known host such as Escherichia coli and Bacillus sublilius by using the above plasmid DNA group to obtain a cDNA library. The transformation may be carried out by known methods.

Process (f) may be carried out in order to select a enzyme being usable in the present invention, i.e. a restriction endonuclease not having a recognition site for digestion in cDNA corresponding to mRNA of a target substance, and having the site in the polylinker. Each process may be carried out by known methods and the method hereinbefore described for the process (a).

Process (g) may be carried out by dividing the transformant group obtained in the process (e) into, for example, several ten groups containing several hundred transformants in each group, and assaying each group on activity to select the desired group, and then subjecting the transformants of the selected group to the same procedure for selection, to obtain finally a single desired clone.

Of the feature of the present invention hereinbefore described, the main feature is to synthesize mRNA derived from a target substance in vitro, applying the character that RNA polymerase of Salmonella typhimurim phage SP6 may specifically link to SP6 phage promoter and may transcribe any nucleotide sequence of DNA at a position downstream from the promoter. For that purpose, a known plasmid DNA may be treated to obtain the plasmid DNA which has a SP6 promoter having the cDNA derived from mRNA of a target substance at a position downstream from the promoter, further having a polylinker at a position downstream from the cDNA. for preparing the end of transcription.

Further, the present invention relates to vector-primer DNA, plasmid DNA and linker DNA which used in the methods for cDNA cloning hereinbefore described. Particularly, it relates to vector-primer DNAs which are characterized of having dT chains at a position upstream from the polylinker, and plasmid DNAs which are characterized of having the HindIII-recognition site at a position upstream from the SP6 promoter, and further linker DNAs which are characterized of having the HindIII-cut end at a position upstream from the SP6 promoter and having the dG chains at a position downstream from it.

The present invention may be illustrated in more detail, for an example of cDNA cloning of IgG$_1$ inducing factor as target substance, which is one of lymphokines being desired to clone cDNA in immunology.

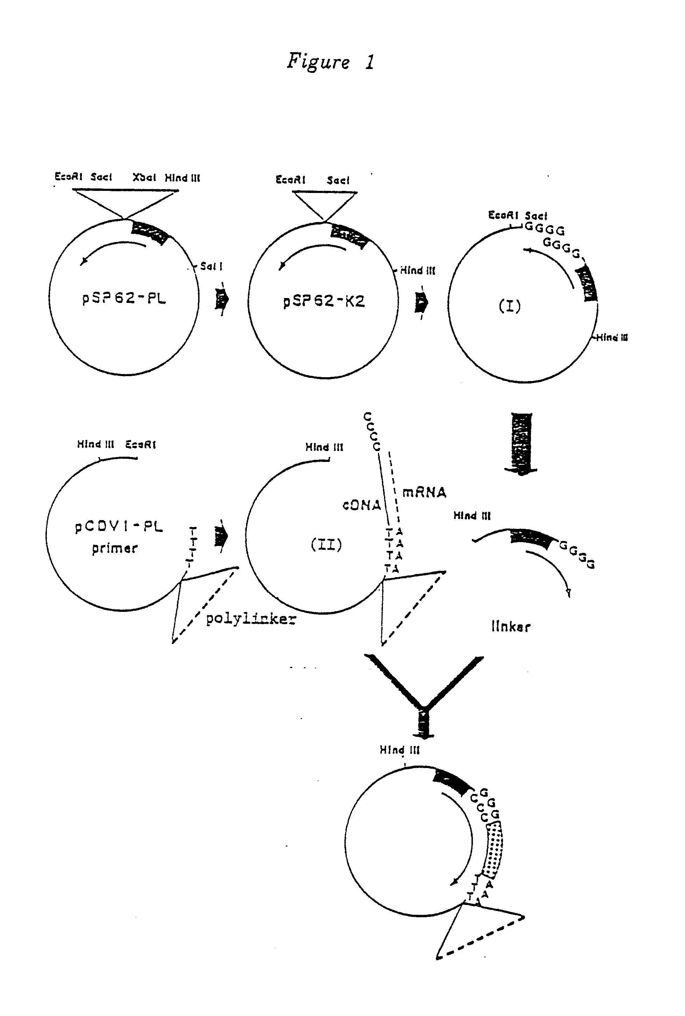

Plasmid DNA pcDV$_1$ (Okayama, H., Berg, P., Mol. Cell. Biol., 3, 280, 1983) is cleaved by restriction endonuclease BamHI and a polylinker is inserted into the BamHI-cut ends thereof to obtain plasmid pcDV$_1$-PL. pcDV$_1$-PL thus obtained is cleaved by restriction endonuclease KpnI and terminal transferase is then allowed to act upon the KpnI-cut ends to add about 50 dT chains. This DNA is further cleaved by restriction endonuclease EcoRI and the larger fragment is collected to obtain the vector-primer DNA (pcDV$_1$-PL primer in Figure 1).

Plasmid DNA pSP-62-PL (being on the market) is cleaved by restriction endonucleases XbaI and HindIII, and both ends are treated with klenow fragment and then cyclized again to prepare the plasmid deficient in SalI-, PstI- and HindIII-recognition sites in the pSP-62-PL polylinker. Further, the SalI-recognition site of the plasmid is replaced by the HindIII-recognition site (pSP-62-K2 in Figure 1). pSP-62-K2 thus obtained is cleaved by restriction endonuclease SacI and terminal transferase is allowed to act upon the SacI-cut ends to add about

3

15 dG chains [(I) in Figure 1]. Then this is cleaved by restriction endonuclease HindIII and the smaller fragment is collected to obtain the linker DNA (linker in Figure 1).

Poly A+RNA separated from T cells (B6.C-H-2bm12 2.19 T cells) secreting IgG1 inducing factor is fractionated by the sucrose gradient method, and then the fraction containing mRNA of IgG1 inducing factor, selected by the assay on the biological activity hereinafter described, is hybridized with the above vector•primer and then a DNA is prepared by reverse transcriptase, using RNA as template. Ten to 15 dC chains are added to the above DNA by terminal transferase, and then the obtained DNA is cleaved by HindIII [(II) in Figure 1]. Next, the linker DNA obtained above is added thereto to cyclize and then the RNA moiety thereof is replaced by DNA (Figure 1). Then, E. coli HB101 is transformed with the cDNA-containing plasmid DNA to give cDNA library.

Plasmid DNA is separated from cDNA library consisting of about 4000 independent transformants, and cleaved by three kinds of restriction endonuclease, i.e. PstI, SacI and SalI to produce a template for in-vitro transcription. The in-vitro transcription is carried out with the action of SP6RNA polymerase to prepare the RNA.

In this case, by adding $m^7G(5')G$ (being on the market) to the substrates, i.e. ATP, CTP, GTP and UTP, the RNA having the Cap structure characteristic of eukaryote mRNA, may be prepared (Konarska, M, M., et al., Cell, 38, 731, 1984). The mRNA prepared by in-vitro transcription is injected into oocytes of Xenopus laevis. After incubation for 36 hours at 20°C, the culture supernatant is examined for IgG1 inducing factor activity by the following procedure. Splenic cells ($4 \times 10^5$ cells/m$\ell$) of mice (female c57 Black, 6- to 10-week age) are suspended in RPMI-1640 containing 15% FCS and $2 \times 10^{-5}$ M 2-mercaptoethanol, and stimulated by 5- μg/m$\ell$ lipopolysaccharide (LPS), and after 24 hours, the culture supernatants of the oocytes sterilized by passing through a filter are added thereto. After incubation for five days, the number of cells capable producing IgG1 and the number of cells capable producing IgG3 are measured by the reverse plaque assay [Gronowicz, E., et al., Eur. J. Immunol., 6, 588, 1976) to examine for IgG1 inducing factor activity. IgG1 inducing factor activity was observed only in the culture supernatant of the oocytes which the RNA prepared by using as template DNA cleaved by SalI, was injected into (Table-1). SalI was found to be the restriction endonuclease suitable for IgG1 inducing factor activity.

Next, groups consisting of small number of transformants are prepared, and plasmid DNA containing cDNA is separated from each group and cleaved by SalI to produce a template for in-vitro transcription, Subsequently, by using the same procedure hereinbefore described, the group having IgG1 inducing factor activity may be selected. The repetition of the same screening finally gives the separation of a single clone.

As explained above, it is demonstrated that the method of the present invention is very effective for cDNA cloning when it is the only method for identifying the target substance to measure their biological activity.

The present method is, of course, effective for cDNA cloning of proteins other than IgG1 inducing factor.

IgG1 inducing factor is secreted in the culture supernatants of the oocytes. If a protein is not secreted in the culture supernatants, the homogenate supernatants of the oocytes may be effectively analyzed because the substance inhibiting the activity scarcely exists in the supernatants of the oocytes generally.

The following examples illustrate, but not limit, the present invention.

[Example 1]

Preparation of cDNA library

(1) Preparation of vector•primer DNA

Plasmid DNA pSP65 (product of Promega Biotec Inc.) was cleaved with restriction endonucleases EcoRI and Hind III to cut out a polylinker DNA, which in turn was treated with Klenow fragment, followed by coupling, at its both ends, of BamHI linker DNA with its 5'-terminal phosphorylated by $T_4$ DNA ligase, and by cleavage with BamHI. giving a polylinker DNA having BamHI-cut terminals. This polylinker DNA may be replaced by a synthetic nucleotide.

Separately, plasmid DNA pcDV1 (Okayama, H., et al., Mol. Cell. Biol., 3, 280, 1983) was cleaved by restriction endonuclease BamHI at 37°C for one hour, 30 μ$\ell$ of the reaction mixture thus obtained [containing 2 μg pcDV1, 10mM Tris-HCl (pH 8.0), 7mM MgCl₂, 2mM 2-mercaptoethanol, 0.01% bovine serum albumin (BSA), and 6 units of BamHI)] was extracted with phenol/chloroform, and ethanol was added to the extract to precipitate DNA.

After being washed with ethanol and dried, the precipitate was dissolved in 100 μ$\ell$ of 10mM Tris-HCl (pH 8.0), 0.4 unit of alkaline phosphatase was added, and the reaction was continued at 60°C for one hour. The reaction mixture was extracted with phenol/chloroform, and ethanol was added to the extract to precipitate DNA. This DNA (1 μg) and the polylinker DNA obtained above (0.5 μg) were allowed to react in a ligation solution [containing 50mM Tris-HCl (pH 7.4), 10mM MgCl₂, 10mM DTT and 1mM ATP)] at 15°C for six hours by 5 units of $T_4$ DNA ligase, affording pcDV1 with the polylinker DNA inserted therein (pcDV1-PL). E. coli HB101 was transformed with pcDV1-PL obtained above by the known technique (Mandel, et al., J. Mol. Biol., 53, 154, 1970), the strains carrying pcDV1-PL were selected from the transformants, and said plasmid DNA was isolated from these selected strains by the usual method.

pcDV1-PL thus prepared (200 μg) and restriction endonuclease KpnI (350 units) were allowed to react in 400 μ$\ell$ of a reaction mixture [containing 6mM Tris-HCl (pH 7.5), 6mM MgCl₂, 6mM 2-mercaptoethanol and 0.02% BSA)] at 37° C for five hours, the reaction mixture was extracted with phenol/chloroform, and DNA thus extracted was reprecipitated twice by addition of ethanol, washed with 70% ethanol and recovered.

Terminal transferase (TTase) was then allowed to act upon the KpnI-cut ends of the DNA obtained above in 200 µℓ of a solution [containing 140mM sodium cacodylate, 30mM Tris-HCl (pH 6.8), 1mM CoCl₂, 0.1mM dithiothreitol (DTT), 0.2mM dTT (containing 4 µCi $^3$H-dTTP) and 108 units of TTase] unit dT chain was added to a 45-base length on average, while monitoring the elongation of dT chain by measurement of the incorporation of $^3$H-dTTP at 10-minute intervals. The reaction was terminated by addition of 20 µℓ of 0.2M EDTA and 10 µℓ of 10% SDS, and the DNA formed was isolated by extraction with phenol/chloroform and purified by precipitation and washing with ethanol.

This DNA, after being treated with 200 units of restriction endonuclease EcoRI at 37°C for five hours, was subjected to electrophoresis on 1% agarose gel, and the larger fragment was collected by the DEAE-paper method (Dretzen, G., et al., Anal. Biochem., 112, 295, 1981). This DNA fragment was dissolved in 400 µℓ of an aqueous solution containing 1M NaCl, 10mM Tris-HCl (pH 7.4) and 1mM EDTA, the resulting solution was cooled to 0° C and absorbed on 0.5 mℓ oligo-dA column previously equlibrated with an aqueous solution of the same composition as above, and the column was washed with 30 mℓ of 1M NaCl-TE solution. The absorbed portion was eluted with 65°C distilled water, the eluate was extracted with phenol/chloroform, and the extracted product was precipitated twice with ethanol, followed by washing with ethanol, giving the objective vector•primer DNA.

## (2) Preparation of linker DNA

Plasmid DNA pSP-62-PL (product of NEN Inc.) was treated in 20 µℓ of a solution containing 3 µg pSP-62-PL, 10mM Tris-HCl (pH 7.5), 7mM MgCl₂, 100mM NaCl, 7mM 2-mercaptoethanol, 0.01% BSA, 40 units of XbaI and 6 units of HindIII at 37° C for two hours to cleave XbaI and HindIII cleavage sites in the polylinker section of pSP-62-PL DNA. After incubation, 4 µℓ of a solution containing dATP, dCTP, dGTP and dTTP (2mM each) and 3 units of klenow fragment were added, and the mixture was held at 20°C for 30 minutes. The reaction mixture was extracted with phenol/chloroform, ethanol was added to the extract to precipitate DNA, and 250 ng of this DNA was allowed to react with 5 units of T₄ ligase in 10 µℓ of a ligation solution [containing 50mM Tris-HCl (pH 7.4), 10mM MgCl₂, 10mM DTT and 1mM ATP] at 15°C for 6 hours to effect cyclization. The circular DNA thus prepared was used for transformation of E. coli HB101. Of the transformants selected by using ampicillin resistance as marker, those strains which contain plasmid DNA deficient in the section from HindIII-recognition site to SalI-recognition site in the pSP-62-PL polylinker were screened out, and said DNA was isolated from these strains by the usual method.

The plasmid DNA thus isolated was treated with restriction endonuclease SalI at 37° C for two hours. 30µℓ of the resulting reaction mixture [containing 2 µg of plasmid DNA, 10mM Tris-HCl (pH 7.5), 7mM MgCl₂, 175mM NaCl, 0.2mM EDTA, 7mM 2-mercaptoethanol, 0.01% BSA and 16 units of SalI] was mixed with 5 µℓ of a solution containing dATP, dCTP, dGTP and dTTP (2mM each) and 3 units of Klenow fragment, the reaction was continued at 20°C for 30 minutes, the reaction mixture was extracted with phenol/chloroform, and ethanol was added to the extract to precipitate DNA.

This DNA (2 µg) and HindIII linker DNA with its 5'-end phosphorylated (0.45 µg) were allowed to react in 20 µℓ of the same ligation solution as used above at 15°C for 20 hours by addition of 20 units of T₄ DNA ligase, and the product was employed to transform E. coli HB101. Of the transformants thus formed, those strains containing plasmid DNA in which SalI-recognition site had been lost and HindIII-recognition site had been newly created were screened out, and said plasmid DNA was isolated from these strains by the known technique (pSP-62-K2 in Figure 1).

pSP-62-K2 (100 µg) and SacI (120 units) were allowed to react in 400 µℓ of a reaction mixture [containing 10mM Tris-HCl (pH 7.5), 7mM MgCl₂ and 7mM 2-mercaptoethanol] at 37°C for 1.5 hours, and the reaction mixture was extracted with phenol/chloroform, and the DNA thus extracted was precipitated by addition of ethanol and washed with ethanol.

The DNA isolated above (100 µg) was allowed to react in a solution [containing 140mM sodium cacodylate, 30mM Tris-HCl (pH 6.8), 1mM CoCl₂, 0.1mM DTT, 0.1mM GTP (containing 2 µCi $^3$H-dGTP) and 27 units of TTase] at 37° C for 20 minutes until dG chain of 14-base length on average was added to the SacI-cut end. The reaction was terminated by addition of 0.2M EDTA and 5 µℓ of 10% SDS, the reaction mixture was extracted with phenol/chloroform, and the DNA thus extracted [(I) in Figure 1] was precipitated by addition of ethanol and washed with ethanol.

The DNA thus isolated was allowed to react in a solution containing 10mM Tris-HCl (pH 7.5), 7mM MgCl₂, 60mM NaCl and 50 units of HindIII at 37°C for two hours, the reaction mixture was subjected to electrophoresis on 1.5% agarose gel, and the smaller fragment (approximately 500 base pairs) was collected by the DEAE-paper method, affording the objective linker DNA.

## (3) Synthesis of cDNA

Five units of reverse transcriptase was added to a mixture of poly A+ RNA (3 µg) [which was isolated from stimulated T cells (B6.C-H-2bm12 2.19 T cells) and then fractionated by the sucrose gradient method] and vector•primer (0.7 µg) prepared in (1) above in 20 µℓ of a solution containing 50mM Tris-HCl (pH 8.3), 8mM MgCl₂, 30mM KCl, 0.3mM DTT, 2mM dNTP (dATP, dGTP, dCTP and dTTP each) and 10 µCi α-$^{32}$p-dCTP, the reaction was continued while monitoring the incorporation of $^{32}$p-dCTP, and the reaction mixture was worked up in the same manner as above (termination, extraction, reprecipitation and washing).

The DNA thus obtained was subjected to the same manner as above for the elongation of dT or dG chain to obtain the DNA to which dC chain of a 14-base length on average was added.

The DNA thus obtained was treated with 5 units of HindIII in a reaction mixture containing 6mM Tris-HCl (pH 7.5), 6mM of MgCl$_2$, 6mM 2-mercaptoethanol, 50mM NaCl and 0.01% BSA at 37°C for one hour, and the reaction mixture was worked up in a similar manner, giving DNA shown in (II) of Figure 1.

This DNA and the linker DNA obtained in (2) above were allowed to react in 100 $\mu\ell$ of a solution [containing 20mM Tris-HCl (pH 7.5), 4mM MgCl$_2$, 10mM (NH$_4$)$_2$SO$_4$, 0.1M KCl, 0.1mM β-NAD, 0.01% BSA and 0.6 μg of E. Coli DNA ligase] overnight at 12°C. The following components were then added at respective specified concentrations [dATP, dCTP, dGTP and dTTP to 40μM each; β-NAD to 0.15mM; E. coli DNA ligase (0.4 unit); E. coli DNA polymerase (0.3 unit); and E. coli RNase H (1 unit)], and the resulting mixture was allowed to react at 12°C for one hour and at 25°C for one hour, affording a solution containing the objective plasmid carrying cDNA. This solution was submitted to the next step without any treatment.

(4) Preparation of cDNA library

Competent cells of E. coli HB101 were transformed with the cDNA-containing plasmid DNA obtained in (3) above by the known technique, giving cDNA library. From this library were separated 16 groups, each composed of 250 independent transformants.

[Example 2]

In-vitro transcription

(1) Preparation of template DNA and selection of restriction endonuclease

Plasmid DNA, isolated from the cDNA library obtained in Example 1 in the same manner as above, was treated with PstI, SacI and SalI each, and the resulting DNAs obtained by extraction with phenol/chloroform, followed by precipitation and washing with ethanol, were used as template in the in-vitro transcription process detailed in (2) below.

The in-vitro transcription and the screening of the IgG$_1$ inducing factor activity, both detailed below were carried out by using three kinds of template DNA obtained above. The result of which is summarized in Table 1. As is apparent from the table, SalI was found to be the suitable restriction endonuclease for the purpose. Hence, the DNA prepared by treatment with this enzyme was adopted as template DNA for in-vitro transcription.

(2) In-vitro transcription

The components given below were quickly mixed in that order, and the mixture was held at 40°C for one hour to complete the reaction.

| 1 | Pure water (distilled twice) | 19.3 $\mu\ell$ |
|---|---|---|
| 2 | Transcription buffer | 10 |
| 3 | 1M DTT | 0.5 |
| 4 | Ribonuclease inhibitor (70 units/$\mu\ell$) | 0.7 |
| 5 | $_\gamma$NTPs | 4 |
| 6 | 10mM m$^7$G(5')ppp(5')G | 5 |
| 7 | 1 mg/m$\ell$ BSA | 5 |
| 8 | Template DNA (1 $\mu$g/$\mu\ell$) | 3 |
| 9 | α-32P-GTP (10000 cpm/$\mu\ell$) | 2 |
| 10 | SP6 RNA polymerase (15 units/$\mu\ell$) | 0.5 |
| | Total: | 50 $\mu\ell$ |

wherein transcription buffer is [200mM Tris-HCl (pH 7.5), 30mM MgCl$_2$ and 10mM spermidine]; $_\gamma$NTPs is [2.5mM ATP, CTP, GTP and UTP]; and m$^7$G(5')ppp(5')G is an analogue of cap structure of eukaryote mRNA (product of P-L Biochemicals) — a compound composed of two units of GTP linked together at 5' ends, with the guanine at one end methylated at 7-position.

The synthetic mRNA thus formed was isolated from the substrate $_\gamma$ NTPs by gel filtration technique as described below.

A disposable syringe (1 m$\ell$ capacity) was charged with Sephadex G-50 (Pharmacia) previously equilibrated with TE buffer [containing 10mM Tris-HCl (pH 8.0) and 1mM EDTA (pH 8.0)]. The reaction mixture obtained

6

above was applied to this column, and the synthetic mRNA was recovered in the eluate after centrifugation at 1300 rpm for four minutes. Counting the $^{32}$P contained in the eluate showed that 1.5 to 2.5 μg of the synthetic mRNA could be recovered.

The crude product thus collected was extracted with phenol, precipitated with ethanol and dried, and the purified product was dissolved in distilled water (o.3 μg/μℓ).

[Example 3]

Screening of IgG$_1$ inducing factor cDNA

mRNAs were prepared from each of the 16 groups of cDNA library obtained in Example 1(4) in the same manner as in Example 2 to obtain 16 groups of mRNA solution (0.3 μg/μℓ).

A mature, female Xenopus laevis (10 cm long) was anesthetized in ice-water and oocytes were taken out from her and separated into individuals in a modified Barth's culture medium (Colman, A., Transcription and Translation --- a practical approach, p291; edited by Hames, B.D. and Higgins, S.J.; IRL Press, 1984) by platinic wire.

Each of the mRNA solutions prepared above (0.3 μg/μℓ) (70 ng) was injected into one piece of oocyte by means of a capillary and micromanipulator while observing under a microscope. Twenty pieces of oocyte were used for each mRNA sample. Each of the treated oocytes were incubated in 10 μℓ of modified Barth's medium per oocyte at 20°C for 36 hours. The supernatant of each culture was centrifuged at 4°C using an Eppendorf centrifuge at 12000 rpm for 20 minutes, and the supernatant was collected and passed through a gas-sterilized Milipore Filter (pore size: 0.45 μm) to obtain the supernatant containing a protein induced by mRNA.

Lymphocytes were collected from the spleen of female C57BL mice (6- to 10-week age) by the usual method, washed twice with RPMI-1640 (containing 2% FCS), and suspended in a medium (RPMI-1640 containing 15% FCS and 5 x 10$^{-5}$M 2-mercaptoethanol) at a cell concentration of 4 x 10$^5$/mℓ. After LPS (Lipopolysaccharide E. coli 0111:B4; Difco Laboratories) was added to a concentration of 50 μg/mℓ, the resulting cell suspension was out on a 96-well microtiterplate (200 μℓ in each well), and after 24 hours, each of the culture supernatants of the treated oocytes prepared above was added to each well (20 μℓ and 50 μℓ in each). After incubation for four to five days, the number of cells capable of producing IgG$_1$ and the number of cells capable producing IgG$_3$ were measured for each well by the reverse plaque assay (Gronowicz, E., et al., Eur. J. Immunol., 6, 588, 1976) with the index of

$$\frac{A}{A + B}$$

(wherein "A" represents the number of cell generating IgG$_1$ and "B" represents the number of cell generating IgG$_3$) to assay IgG$_1$ inducing factor activity. High IgG$_1$ inducing factor activity was observed in three groups (#1, #3 and #16) of 16 groups.

The cDNA plasmid of two groups (#1 and #3) out of the three groups having the inducing factor activity was used to transform E. Coli HB101, 400 clones were randomly picked out, and each of these was cultured in 0.5 mℓ of L broth (Luria's medium) containing 30 μg/mℓ ampicilin (total 800 clones: 400 clones were picked out from each group of #1 and #3). Each 20 μℓ of culture was collected from 25 clones of one group and was mixed together. By this procedure, 32 groups were prepared from two groups (#1 and #3). In the same screening as hereinbefore described, high IgG$_1$ inducing factor activity was observed in three groups (#1-4, #3-12 and #3-15) of 32 groups. Furthermore, in the same screening of all clones (2 x 25) of two groups (#3-12 and #3-15) as above, high IgG$_1$ inducing factor activity was observed in two clones.

## Table 1   IgG1 Inducing Factor Activity by
## Culture of Mouce Splenic Lymphocytes

| Additive to lymphocyte culture | % | DNA used as template | Number of plaqueforming cells in culture | |
|---|---|---|---|---|
| | | | IgG1 | IgG3 |
| Oocyte culture supernatant | 10 | DNA obtained by cutting cDNA with SalI | 175 | 218 |
| | 20 | | 490 | 396 |
| Oocyte culture supernatant | 10 | DNA obtained by cutting cDNA with SacI | 12 | 110 |
| | 20 | | 53 | 218 |
| Oocyte culture supernatant | 10 | DNA obtained by cutting cDNA with PstI | 17 | 898 |
| | 20 | | 17 | 370 |
| Phosphate buffer | 10 | None | 22 | 257 |
| | 20 | | 43 | 658 |
| None | 0 | None | 17 | 749 |
| | 0 | | 26 | 782 |
| B6.C-H-2bm12 2.19 T cells | 5 | None | 1516 | 101 |

BRIEF DESCRIPTION OF THE DRAWING

Figure 1 is a diagram illustrating plasmid DNA construction in the method of the present invention.

**Claims**

1. A method for cDNA cloning, which is characterized by:

(a) isolating and fractionating a mRNA group containing mRNA of a target substance in a conventional method.

(b) hybridizing the mRNA group with a vector•primer DNA containing a dT chain at a position upstream from a polylinker, obtained from a known plasmid DNA,

(c) synthesizing a single-chained cDNA by using reverse transcriptase, and then inserting a deoxyribonucleotide chain at the end to obtain a vector DNA,

(d) linking up the vector DNA with a linker DNA which has a SP6 promoter followed by the deoxyribonucleotide chain which can make a pair with the deoxyribonucleotide chain inserted in the process (c), at a position downstream from the promoter, obtained from a known plasmid DNA, and then replacing mRNA by DNA to obtain a plasmid DNA group corresponding to each mRNA in mRNA groups,

(e) transforming the host by using the plasmid DNA group thus obtained,

(f) separating the plasmid DNA group from the transformant, subjecting to in-vitro transcription using SP6 RNA polymerase and using as template, the cDNA group which is obtained by digesting with each of some kinds of restriction endonuclease, to synthesize mRNA groups, translating the mRNA group to obtain protein groups, and then assaying the protein groups on activity of the target substance to select a proper restriction endonuclease, and

(g) subjecting each groups containing a small number of the transformants obtained in the process (e), to the same procedure as the process (f) by using the restriction endonuclease selected in the process (f) to select an active group, and then subjecting the transformants of the selected group to the same procedure.

2. A vector•primer DNA which is characterized of having dT chains at a position upstream from a polylinker

3. A vector-primer DNA as claimed in claim 2, which is characterized of having dT chains at a position a long way upstream from a polylinker.

4. A vector-primer DNA as claimed in claim 2 or 3, wherein the dT chains comprise about 50 bases.

5. A vector-primer DNA as claimed in claim 2, 3 or 4, which is pcDV1-PL.

6. A plasmid DNA which is characterized of having the HindIII-recognition site at a position upstream from a SP6 promoter.

7. A plasmid DNA as claimed in claim 6, which is pSP62-K2.

8. A linker DNA which is characterized of having the HindIII-cut end at a position upstream from a SP6 promoter and having the dG chains at a position downstream from it.

9. A linker DNA as claimed in claim 8, which has the dG chains of about 15 bases at a position a long way downstream from a SP6 promoter.

Claims for the following Contracting State: AT; ES; GR

1. A method for cDNA cloning, which is characterized by:

(a) isolating and fractionating a mRNA group containing mRNA of a target substance in a conventional method,

(b) hybridizing the mRNA group with a vector•primer DNA containing a dT chain at a position upstream from a polylinker, obtained from a known plasmid DNA,

(c) synthesizing a single-chained cDNA by using reverse transcriptase, and then inserting a deoxyribonucleotide chain at the end to obtain a vector DNA,

(d) linking up the vector DNA with a linker DNA which has a SP6 promoter followed by the deoxyribonucleotide chain which can make a pair with the deoxyribonucleotide chain inserted in the process (c), at a position downstream from the promoter, obtained from a known plasmid DNA, and then replacing mRNA by DNA to obtain a plasmid DNA group corresponding to each mRNA in mRNA groups,

(e) transforming the host by using the plasmid DNA group thus obtained,

(f) separating the plasmid DNA group from the transformant, subjecting to in-vitro transcription using SP6 RNA polymerase and using as template, the cDNA group which is obtained by digesting with each of some kinds of restriction endonuclease, to synthesize mRNA groups, translating the mRNA group to obtain protein groups, and then assaying the protein groups on activity of the target substance to select a proper restriction endonuclease, and

(g) subjecting each groups containing a small number of the transformants obtained in the process (e), to the same procedure as the process (f) by using the restriction endonuclease selected in the process (f) to select an active group, and then subjecting the transformants of the selected group to the same procedure.

2. A method of obtaining a vector-primer DNA comprising cleaving a DNA molecule and inserting chains at a position upstream from a polylinker.

3. A method of obtaining a plasmid DNA comprising inserting into A DNA molecule. a HindIII-recognition site at a position upstream from a SP6 promoter.

4. A method of obtaining a linker DNA comprising cleaving a DNA molecule to give a HindIII-cut end at a position upstream from SP6 promoter and having dG chains at a position downstream from it.

Figure 1